# FASCICULE DE BREVET EUROPEEN

(11) **EP 0 310 507 B1**
(45) Date de publication et mention de la délivrance du brevet: **26.10.1994**
(21) Numéro de dépôt: 88402455.5
(22) Date de dépôt: 28.09.1988
(51) Int. Cl.: A45D 34/00, A45D 40/00

(54) **Nouveau dispositif destiné à conserver les substances cosmétiques congelables**
Vorrichtung zum Aufbewahren von gefrierbaren kosmetischen Erzeugnissen
Device for preserving congealable cosmetic products

(30) Priorité: 30.09.1987 FR 8713504
(43) Date de publication de la demande: 05.04.1989
(73) Titulaire: Bontemps, Raymond, F-75016 Paris (FR)
(72) Inventeur: Bontemps, Raymond, F-75016 Paris (FR)
(74) Mandataire: Gutmann, Ernest

(56) Documents cités:
- EP-A- 0 017 595
- FR-A- 2 160 285
- FR-A- 2 261 931
- FR-A- 2 410 474
- FR-A- 2 555 446
- US-A- 4 029 112

## Description

La présente invention concerne un coffret renfermant un étui plastique cylindrique ou tronconique fermé par un couvercle étanche , contenant une substance cosmétique déshydratée par lyophilisation. Associées à cet étui, on trouve deux ampoules de sérum ou autre solution placées dans le coffret. L'ensemble substance cosmétologique et sérum physiologique est mélangé à température ambiante, puis congelé à -30°C avant d'etre appliqué sur la peau afin d'obtenir un traitement cryogénique de la peau .

On emploie actuellement de nombreux cosmétiques frais d'origine vivante exempt de tout excipient et conservateur formé d'une substance foetale tels que la gelée de WARTON, le mesenchysme, le cartilage de Meekel, le thymus, l'amnios, utilisés purs ou en association . Ces produits sont généralement prélevés sur un foetus animal, broyés et réduits en une pâte homogène. Cette dernière est ensuite diluée dans du sérum physiologique et conditionnée sous forme de pain solide à -80°C.

Ces produits, souvent d'origine foetale, composés de biogènes ou cellules spécifiques à base d'ADN ou d'ARN, sont supposés régénérer la matière vivante de la peau.

Pour conserver leur caractère actif ils sont conservés à l'état congelé voisin de la température de - 80°. Cette conservation nécessite des dispositifs cryogéniques importants, notamment durant leur transport. Les caractéristiques de régénération nécessitent qu'ils soit utilisés à l'état frais. Cette nécessité de maintenir les substances foetales à basse température permet également en cosmétologie d'utiliser les effets de la cryogénie sur la peau.Par exemple massage de la peau du visage à basse température au moyen de ces substances foetales , broyées et purifiées, permet de provoquer d'une part, un effet vaso-constricteur de la peau entraînant les rejets des particules inertes ou graisseuses contenant dans les pores.

Le réchauffement qui se produit avec l'application de la substance foetale congelée va provoquer l'absorption de ces substances omniprésentes par le massage sur la peau. Ce phénomène a pour objet de régénérer les tissus de l'épiderme.

Pour conserver cet avantage tout en manipulant les substances foetales dans les meilleures conditions de transport sans avoir à les maintenir dans une enceinte à - 80°, on a imaginé de les purifier, les centrifuger rapidement après leur prélèvement et les lyophiliser pour les débarrasser toute substance susceptible de les altérer. On obtient une substance foetale séchée par lyophilisation que l'on hydrate au moment de l'utilisation avec du sérum physiologique.

En outre, le mélange substance foetale et sérum physiologique constitue une pâte qui est congelée à une température plus faible de moins 30° : ceci afin d'obtenir un bâtonnet solidifié qui présente, lors de l'utilisation, des avantages de la cryogénie utilisée par les cosmétologues et la propriété de régénérer la peau.

Pour faciliter la manipulation de ces bâtonnets et limiter le réchauffement par l'utilisation de la main, ces derniers sont pourvus d'un manche plastique ou de bois non conducteur de la chaleur. La protection de ces bâtonnets est assurée par un étui plastique qui vient s'adapter sur une rondelle de dimensions appropriées solidaire d'une manche afin d'obtenir une "sucette".

La présente invention dénommée "Dispositif destiné à conserver les substances cosmétiques congelables d'origine foetale", est caractérisée en ce qu'il comprend un coffret comprenant deux ampoules de sérum physiologique, un étui contenant la substance cosmétologique d'origine foetale purifiée, lyophilisée et séchée ; cette substance est placée dans un étui durant son transport, doté d'un bouchon plastique vissé, l'étui étant étanche avant l'utilisation, deux orifices peuvent être percés au moment de la première utilisation afin d'introduire le sérum physiologique, ce bouchon porte une couronne plastique contenant la substance foetale lorsqu'elle est imbibée de sérum physiologique, puis congelée avant sa première utilisation. Un languette constitue un moyen de préhension manuel externe.

L'invention ainsi caractérisée présente de nombreux avantages et notamment :
- une simplification de stockage qui peut être réalisée à température ordinaire.
- une souplesse de manipulation,
- une bonne conservation de la substance foetale,
- une économie de matière active.

L'invention sera mieux comprise grâce aux dessins annexés qui ne sont présentés que comme une réalisation préférentielle, notamment en ce qui concerne la forme, le volume et la nature des matériaux utilisés.

La figure 1 représente le coffret ouvert où sont logés l'étui et les ampoules de sérum physiologique.

La figure 2 représente l'étui et la figure 3 représente le bâtonnet de substance congelé porté par son support.

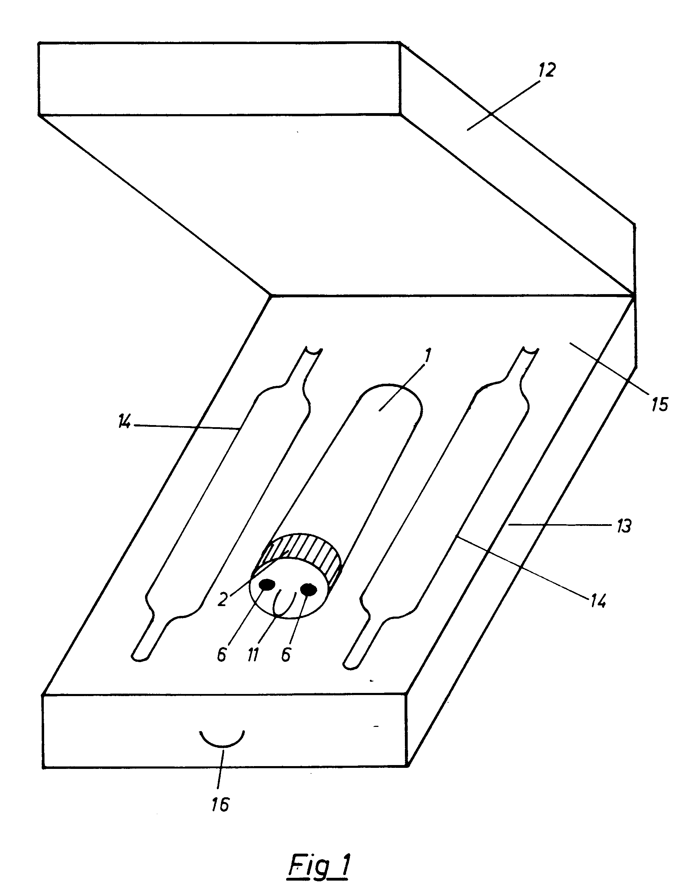

Suivant une caractéristique importante de l'invention, on a représenté sur la figure 1 le coffret (13) fermé par un couvercle (12) . A l'intérieur de ce coffret, on trouve l'étui plastique contenant la substance foetale déshydratée. Cet étui est fermé à sa base par un bouchon plastique (2) qui s'emboîte parfaitement sur l'étui. Sur ce bouchon, on trouve les orifices (6) utilisés pour le remplissage de l'étui par du sérum physiologique. Un moyen de préhension isotherme (11) permet la manipulation du bâtonnet (4).
De chaque côté de l'étui (1) on a logé 2 ampoules de sérum physiologique (14) . L'ensemble étui et ampoules de sérum est logé dans un lit de mousse expansée (15) afin d'éviter les chocs durant les transports . Lorsque le coffret est fermé , il peut être aisément manipulé par la poignée (16) .

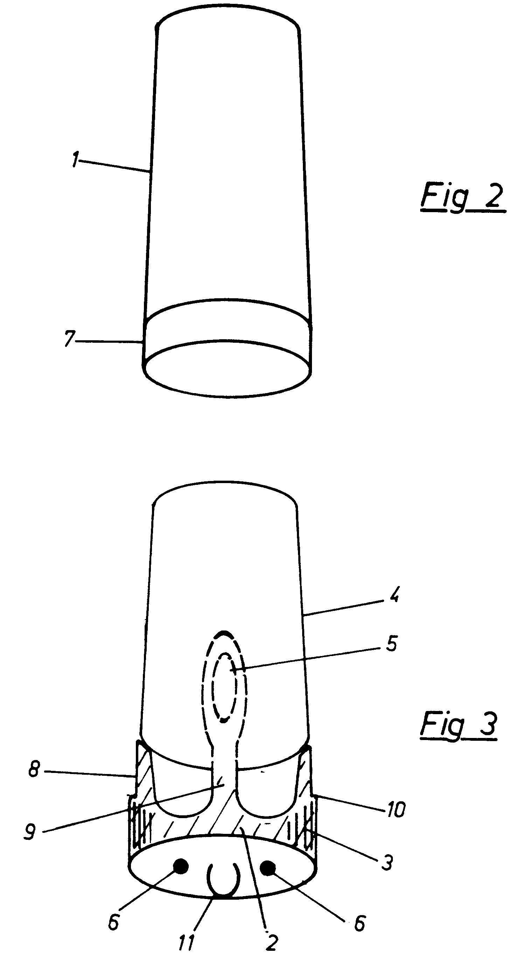

On représente sur les figures 2 et 3 l'étui et le bouchon portant la substance foetale ou cosmétique .

L'étui en plastique (1) ; de forme tronconique, a une base (7) de section cylindrique, qui s'emboîte parfaitement sur le bouchon au niveau d'un support (3) et d'une couronne.

Le bouchon représenté sur la figure 3 est constitué d'un socle (2) portant une couronne (8) servant à l'emboîtement de l'étui.
L'épaulement (10) assure l'étanchéité entre l'étui (1) et le bouchon (3). Une languette (5) qui prend naissance à partir du bouchon, émerge et supporte la substance foetale ou cosmétique (4) lorsque celle-ci, imbibée de sérum physiologique, est congelée.
Les orifices (6) servent au remplissage de l'étui en sérum ou soluté. La languette (11) permet la préhension de l'ensemble.
Les stries de la couronne (2) facilitent le déboîtage étui-bouchon. Les éléments 2, 5, 8 sont réalisés en plastique moulé et forment un bloc unique.

UTILISATION : L'étui préalablement rempli de substance foetale déshydratée par lyophilisation est transporté à température ordinaire. Lorsque l'on désire effectuer une application cosmétologique, l'étui contenant la substance est rempli de sérum en introduisant ce dernier par l'un des orifices (6) et placé dans un congélateur à - 30° C. Après congélation, l'étui est séparé par déboîtage du support (3) et l'on peut pratiquer le traitement par application sur la peau.

## Revendications

1. Dispositif destiné à conserver les substances cosmétiques congelables d'origine foetale, caractérisé en ce qu'il comprend un coffret comprenant deux ampoules de sérum physiologique (14), un étui (1) contenant la substance d'origine foetale (4), purifiée, lyophilisée et séchée ; cette substance est placée dans l'étui (1) durant son transport, doté d'un bouchon de plastique vissé (2), l'étui étant étanche avant l'utilisation, deux orifices (6) peuvent être percés au moment de la première utilisation afin d'introduire le sérum physiologique, ce bouchon porte une couronne plastique (8) soutenant la substance foetale lorsqu'elle est imbibée de sérum physiologique puis congelée avant utilisation, une languette (11) constitue un moyen de préhension manuel externe.

2. Dispositif selon la revendication 1, caractérisé en ce que le remplissage de l'étui (1) contenant la substance foetale est obtenu au moyen de sérum physiologique introduit dans les orifices (6), situés sur le bouchon (2) plastique qui se visse à la base (7) de l'étui tronconique.

3. Dispositif selon la revendication 1, caractérisé en ce que la substance foetale congelée constitue, lorsqu'elle est imbibée de sérum physiologique contenue dans les ampoules (14), un bâtonnet (4) porté au moyen d'une languette (5) plastique collé sur le bouchon (2) l'étui protecteur étant déboîté du support (3) pour faire apparaître le bâtonnet congelé (4).

4. Dispositif selon la revendication 4, caractérisé en ce que la substance foetale congelée est utilisée comme élément de massage sur la peau, au moyen du bâtonnet (4) composé de substances foetales et de sérum physiologique provenant des ampoules (14) et porté par le support (3) complété par une couronne (8) utilisés comme logement-support du bâtonnet (4) ; une languette (11) extérieure permet la préhension manuelle sans réchauffement.

## Claims

1. A device used for the preservation of freezable embryo extracts for use in cosmetics, characterized by a box containing two vials of saline solution (14), a sheath (1) containing the embryo extracts (4) which have been purified and freeze-dried; the extracts are placed in the sheath (1) during transportation, which has a screw-on plastic cap (2), the sheath being air and waterproof before use, with two openings (6) that can be punctured in order to introduce the saline solution at the time of first utilization, this cap has a plastic ring (8) which holds the embryo extracts when they are soaked in saline solution then frozen before use, a small exterior tab (11) allows external handling.

2. A device according to Claim 1, whereby the sheath (1) containing the embryo extracts can be filled by means of pouring the saline solution through the openings (6), located on the plastic cap (2) which is screwed onto the base (7) of the sheath shaped like a truncated cone.

3. A device according to Claim 1, whereby the frozen embryo extracts constitute, when they are soaked in saline solution contained in the vials (14), a stick (4) held by means of a plastic tab (5) stuck to the cap (2), with the protective sheath being taken out of its base (3) in order to expose the frozen stick (4).

4. A device according to Claim 1, whereby the frozen embryo extracts are used for skin massages, by means of the stick (4) made up of the embryo extracts and the saline solution from the vials (14) and held by the base (3) complemented by a ring (8) which is used as the container and support of the stick (4); a small exterior tab (11) serves to hold and handle the product without heat transfer.

## Patentansprüche

1. Vorrichtung zur Konservierung tiefkühlbarer Substanzen fötaler Herkunft für den Gebrauch im Kosmetikbereich, dadurch gekennzeichnet, daß sie aus einem Behälter mit zwei physiologische Kochsalzlösung (14) enthaltenden Ampullen, sowie einem Etui (1) besteht, welches die reine, gefriergetrocknete Substanz fötaler Herkunft(4) beinhaltet: diese Substanz wird während des Transports in das vorgenannte Etui (1)eingebracht, welches mit einem Kunststoffschraubverschluß(2) versehen ist (das Etui ist hermetisch verschlossen) und zwei Öffnungen(6) aufweist, welche vor dem Erstgebrauch zum Einfüllen der physiologischen Kochsalzlösung durchstoßen werden können, wobei der Verschluß zusätzlich mit einem Kunststoffring (8) ausgestattet ist, der die fötale Substanz hält, während sie mit der physiologischen Kochsalzlösung getränkt und tiefgefroren wird, zudem erlaubt eine kleine Lasche(11) eine Handhabung von außen.

2. Vorrichtung nach Anspruch 1, dadurch gekennzeichnet, daß das Etui (1), welches die fötale Substanz beinhaltet, aufgrund der beiden, im Kunststoffverschluß (2) befindlichen Öffnungen(6), mit der Kochsalzlösung aufgefüllt werden kann, wobei dieser Verschluß am Boden (7) des kegelstumpfförmigen Etuis angeschraubt wird.

3. Vorrichtung nach Anspruch 1, dadurch gekennzeichnet, daß die tiefgefrorene fötale Substanz, wenn sie mit der in den Ampullen (14) befindlichen physiologischen Kochsalzlösung getrankt ist, sich in Form eines Stäbchens(4) darstellt, welches mit Hilfe einer Kunststofflasche (5) gehalten wird, die am Verschluß(2) aufgeklebt ist, wobei das Schutzetui von seinem Unterbau(3) herausgenommen sein muß, damit das tiefgefrorene Stäbchen(4) sichtbar wird.

4. Vorrichtung nach Anspruch 1, dadurch gekennzeichnet, daß die gefrorene fötale Substanz zu Hautmassagen benutzt wird, unter Zuhilfenahme des Stäbchens(4), welches aus fötaler Substanz und der aus den Ampullen(14) stammenden physiologischen Kochsalzlösung zusammengesetzt und auf einen Untersatz(3) montiert ist sowie durch einen Ring (8) ergänzt wird, der dem Stäbchen Halt bietet und gleichzeitig Behälter (4) ist, wobei eine kleine, außen angebrachte Lasche (11), eine Handhabung des Produktes ohne Wärmeübertragung erlaubt.
